(19) Europäisches Patentamt
European Patent Office
Office européen des brevets

(11) Publication number: **0 439 404 B1**

(12) **EUROPEAN PATENT SPECIFICATION**

(45) Date of publication of patent specification: **25.10.95**

(51) Int. Cl.6: **C07D 333/20**, C07D 495/04

(21) Application number: **91400147.4**

(22) Date of filing: **23.01.91**

(54) **Preparation of 2-(2'-thienyl) alkylamines and derivatives thereof and synthesis of 4,5,6,7- thieno [3,2-c] pyridine derivatives therefrom.**

(30) Priority: **25.01.90 US 470018**
**25.01.90 US 470299**

(43) Date of publication of application:
**31.07.91 Bulletin 91/31**

(45) Publication of the grant of the patent:
**25.10.95 Bulletin 95/43**

(84) Designated Contracting States:
**AT BE CH DE DK ES FR GB GR IT LI LU NL SE**

(56) References cited:
**EP-A- 0 342 118**
**FR-A- 2 299 332**
**FR-A- 2 300 090**
**FR-A- 2 614 619**

**CHEMICAL ABSTRACTS, vol. 37, no. 6, 20 March 1943, Columbus, OH (US); G.S. MARKOVA et al., no. 1321**

(73) Proprietor: **SANOFI**
**32-34, rue Marbeuf**
**F-75008 Paris (FR)**

(72) Inventor: **Khatri, Hiralal N.**
**638 West Lilac Court**
**Louisville, CO 80027 (US)**
Inventor: **DeHoff, Bradley S.**
**4785 F Whiterock Circle**
**Boulder, CO 80301 (US)**

(74) Representative: **Polus, Camille et al**
**c/o Cabinet Lavoix**
**2, Place d'Estienne d'Orves**
**F-75441 Paris Cedex 09 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 439 404 B1

## Description

## BACKGROUND OF THE INVENTION

### Field of the Invention

The present invention relates to processes for the synthesis of 2-(2′-thienyl)alkylamines and specifically to an improved process for the conversion of suitably functionalized derivatives of 2-(2′-thienyl)ethanol to 2-(2′-thienyl)ethylamine. It further relates to carbamate salts of 2-(2′-thienyl)alkylamines and specifically to 2-(2′-thienyl)ethylamine useful in the synthesis of 4,5,6,7-thieno[3,2-c] pyridine derivatives, particularly ticlopidine derivatives.

### Background to the Invention

Previous methods for the preparation of 2-(2′-thienyl)ethylamine have suffered from several disadvantages, including low yields (e.g., where the reactions resulted in mixtures of undesirable side products) and high cost.

For example, Braye, U.S. Patent No. 4,128,561 describes a two-step process for making 2-(2'-thienyl)-ethylamine by converting a 2-(2'-thienyl)ethanol to N-2-(2'-thienyl)ethyl phthalimide, and then treating the phthalimide with diethylenetriamine to form the amine. Braye also describes the amination of 2-(2'-thienyl)-alkyl sulfonates with ammonia at elevated temperature and pressure. Braye discloses problems encountered in the preparation of primary amines with ammonia, i.e., the tendency for the process to form secondary and tertiary amines as side products.

A process where 2-(2'-thienyl)ethylbromide is treated with alcoholic ammonia at ordinary temperature for 8 days to produce 2-(2'-thienyl)ethylamine is described by Blicke, et al., J. Am. Chem. Soc., 64, 3, 477-480 (1942).

FR 2 299 332 discloses a process for the preparation of thienyl derivatives of the formula

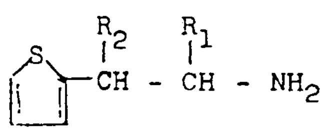

through amination of the corresponding compound of the formula

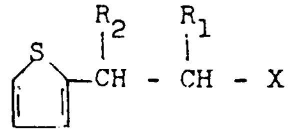

wherein X represents halogen or $-OSO_3R_3$.

For the amination step, this document prescribes (i) the use of ammonia, the reaction being optionally carried out by heating in an autoclave, or (ii) the synthesis of a phtalimide intermediate.

Generally, the synthesis of primary amines with ammonia has been found to be disadvantageous because primary amines are more basic than ammonia; this will cause the primary amine to preferentially attack the reaction substrate over the ammonia, resulting in the formation of secondary and tertiary amines. Advanced Organic Chemistry, March, J., 2nd Ed., 376, (1977). Such formation of undesired secondary and tertiary amines, of course, reduces the yield of the desired primary amine and creates the need for additional purification and isolation steps, which further decrease the yield and increase the cost of the desired primary amine. The formation of undesired secondary and tertiary amines has remained a problem until the present invention.

Other synthetic approaches to making 2-(2'-thienyl)ethylamine have been disclosed in the art, for example, as described below.

The reduction of 2-(2'-thienyl)acetamide with a hydride, e.g., lithium aluminum hydride to form 2-(2′-thienyl)ethylamine is described in Japanese Kokai J6 1221-184-A.

The electrochemical reduction of 2-nitro-2-vinyl thiophene to 2-amino-2-ethyl-thiophene is described in UK Patent Application GB 2,013,196A.

The catalytic hydrogenation of thienyl acetonitrile to form thienyl ethylamines is described in European Patent No. 274,324.

The reduction of nitrovinyl thiophenes with a complex metal hydride, e.g., lithium aluminum hydride to form thiophene ethylamines is described in J. Heterocyclic Chem., 7, 1257-1268 (1970).

The reduction of arylacetonitriles with lithium aluminum hydride/aluminum chloride to form the corresponding 2-aryl-1-aminoethanes is described in Synthesis, 1, 40-42, (1987).

In addition to the yield, side product and cost factors yet another problem encountered with 2-(2′-thienyl)alkylamines in general and 2-(2′-thienyl)ethylamine in particular is their instability when subjected to an oxygen containing atmosphere. This can lead to significant loss of compound to oxidation during storage resulting in increased production cost due to additional purification steps, lower yields, etc.

Ticlopidine

Ticlopidine is a compound with desirable blood platelet aggregation inhibition qualities. Previous technology for the preparation of ticlopidine has entailed a low yielding, labor intensive process, employing certain potentially hazardous and expensive materials. The cost of preparing ticlopidine has, therefore, been high. It has been desired to provide improved synthetic process technology that allows for a higher conversion, reduced labor usage, and the elimination of costly, potentially dangerous materials.

A variety of synthetic approaches to making ticlopidine have been described in the art, including improvements on the various steps of such synthetic processes, e.g., as described below.

Ticlopidine was first described by Castaigne in U.S. Patent No. 4,051,141, where the synthesis thereof was accomplished by condensation of a thieno[3,2-c]pyridine with 2-chlorobenzyl chloride.

One desirable method of preparing ticlopidine calls for 2-(2'-thienyl)ethylamine as a key intermediate. The method involves preparation of a 2-(2'-thienyl)ethanol, conversion to the corresponding sulfonate derivative and then to the amine, followed by benzylation with 2-chlorobenzylchloride and cyclization to give ticlopidine free base, as described by Braye in U.S. Patent No. 4,127,580.

EPA 0342118 pertains to a process for making 2-(2-thienyl) ethylamine via the reduction of 2-(nitrovinyl)-thiophene. This document further teaches the synthesis of ticlopidine starting from the resulting 2-(2-thienyl) ethylamine.

## SUMMARY OF THE INVENTION

One aspect of the present invention relates to a process in which ammonia gas is reacted with a suitably functionalized derivative of 2-(2'-thienyl)ethanol, preferably a halide, alkyl sulfonate or aryl sulfonate in the presence of a metal salt, to form 2-(2'-thienyl)ethylamine, the compound of Formula I.

In another aspect, the invention relates to a process in which liquid ammonia is reacted with a suitably functionalized derivative of 2-(2'-thienyl)ethanol, preferably a halide, aklyl sulfonate or aryl sulfonate in a ketonic solvent, preferably an alkyl ketone or aryl ketone, to form 2-(2'-thienyl)ethylamine, the compound of Formula I.

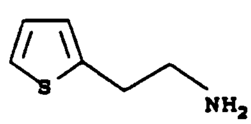

Formula I

The processes for making 2-(2′-thienyl)ethylamine comprise reacting a compound of the formula

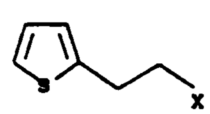

wherein
X is a leaving group,

a) with gaseous ammonia in the presence of a compound of the formula M-Y,
wherein:
M is Na, K, Li, Mg, Zn; and
Y is halo or $CO_3^{-2}$; or

b) with liquid ammonia, and an alkyl ketone or aryl ketone.

In another aspect, the invention relates to a process for the preparation of the carbamate salts of 2-(2'-thienyl)-ethylamine of Formula II. This process is useful for the purification and stabilization of 2-(2'-thienyl)-ethylamine .

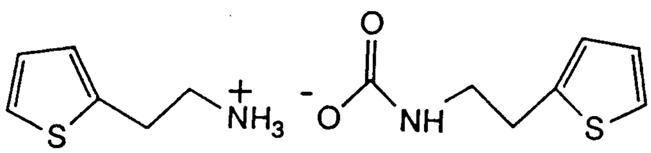

Formula II

The process for making a carbamate salt of a 2-(2'-thienyl)alkylamine of Formula II comprises the steps of

a) reacting a 2-(2'-thienyl)alkylamine, dissolved in an inert solvent, with $CO_2$; and

b) isolating the carbamate salt of the 2-(2'-thienyl)alkylamine.

In another aspect, the invention relates to a process for the synthesis of ticlopidine comprising the steps of

a) reacting 2-(2'-thienyl)ethylamine of the formula

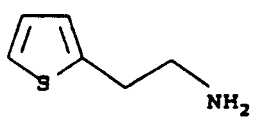

with formaldehyde ($CH_2O$) to give the formimine of the formula

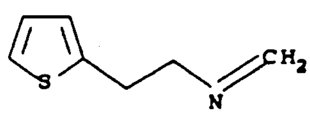

b) cyclizing the formimine by reacting it with aqueous hydrochloric acid to form 4,5,6,7-tetrahydro-thieno-[3,2-c]pyridine of the formula

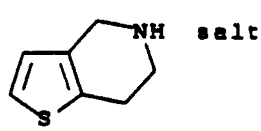

c) alkylating 4,5,6,7-tetrahydrothieno[3,2-c]pyridine by reacting it with 2-chlorobenzyl chloride under either:

(i) phase transfer conditions, or

(ii) by reflux with a base

to give ticlopidine free base of the formula

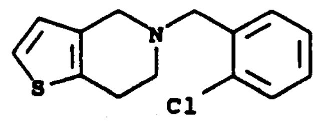

and

d) converting ticlopidine free base to an acid addition salt, preferably the hydrochloride salt.

In still another aspect, the invention relates to a process for the synthesis of ticlopidine comprising the steps of

a) reacting 2-(2′-thienyl)ethylamine with 2-chlorobenzyl chloride to give N-(2-chlorobenzyl)-2-(2′-thienyl)-ethylamine;

b) cyclizing N-(2-chlorobenzyl)-2-(2′-thienyl)-ethylamine by reacting it with formaldehyde to form ticlopidine free base; and

c) converting ticlopidine free base to an acid addition salt, preferably the hydrochloride salt.

In still another aspect, the invention relates to a process for the synthesis of ticlopidine hydrochloride (shown as Formula IV, a salt within the scope of Formula III) from the 2-(2′-thienyl)ethylamine so-prepared.

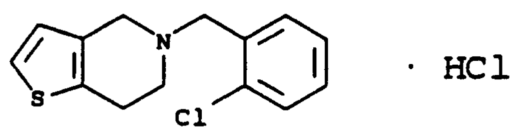

Formula IV

## DETAILED DESCRIPTION OF THE INVENTION

### Definitions and General Parameters

The following definitions are set forth to illustrate and define the meaning and scope of the various terms used to describe the invention herein.

A "pharmaceutically acceptable acid addition salt" of the 4,5,6,7-thieno[3,2-c]pyridine derivatives may be formed with an inorganic or organic acid. The term "pharmaceutically acceptable anion" refers to the anion of such acid addition salts. The salt and/or the anion are chosen not to be biologically or otherwise undesirable. The anions are derived from inorganic acids, such as hydrochloric acid, hydrobromic acid, sulfuric acid (giving the sulfate and bisulfate salts), nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, salicylic acid, methanesulfonic acid, ethanesulfonic acid, p-toluensulfonic acid and the like.

As used herein "alkyl" refers to straight, branched, or cyclic saturated hydrocarbon radicals having from one to six carbon atoms, e.g., methyl, ethyl, n-propyl, isopropyl, n-butyl, isobutyl, t-butyl, cyclopropylmethyl, pentyl, hexyl, cyclohexyl, and the like.

As used herein "alkoxy" refers to the group R-O-, wherein R is "alkyl" as defined above. Examples include methoxy, ethoxy, propoxy, iso-propoxy, iso-butoxy, tert-butoxy, and the like.

As used herein "aryl" refers to a substituted or unsubstituted monovalent unsaturated radical, for example, phenyl or naphthyl, which, if substituted, can have one or more alkyl, alkoxy, hydroxy or halo groups in any available position on the ring.

As used herein "phenyl" refers to phenyl radicals optionally substituted in any available position on the ring with alkyl, alkoxy, hydroxy, or halo.

As used herein "optional" or "optionally" means that the subsequently described event or circumstance may or may not occur, and that the description includes instances where said event or circumstance occurs and instances in which it does not. For example, "optionally substituted phenyl" means that the phenyl may or may not be substituted and that the description includes both unsubstituted phenyl and substituted phenyl.

As used herein "benzyl" refers to the monovalent radical $C_6H_5CH_2$-.

As used herein "benzoyl" refers to the monovalent radical $C_6H_5C(O)$-.

As used herein, the term "halo" or "halogen" refers to fluoro, bromo, chloro and iodo.

As used herein "sulfonate" refers to sulfonic (acid) esters of the formula $RSO_2OR'$ wherein both R and R′ may be alkyl or aryl.

As used herein, the term "alkyl ketone" refers to a ketonic solvent where the carbonyl group is connected to alkyl radicals, i.e., a fully saturated monovalent radical containing only carbon and hydrogen, e.g., methyl, ethyl, propyl, butyl, pentyl, hexyl, and heptyl; and which may be cyclic, branched or straight chain. This term is further exemplified by acetone, methylethyl ketone, diethyl ketone and cyclohexanone.

As used herein, the term "aryl ketone" refers to a ketonic solvent where the carbonyl group is connected to at least one aryl radical, i.e., a monovalent unsaturated aromatic carbocyclic radical having a single ring, e.g., phenyl; or two rings, e.g., naphthyl. This term is further exemplified by diphenyl ketone and methylphenyl ketone.

As used herein, the terms "inert organic solvent" or "inert solvent" mean a solvent inert under the conditions of the reaction being described in conjunction therewith [including, for example, benzene, toluene, acetonitrile, tetrahydrofuran ("THF"), dimethylformamide ("DMF"), chloroform, methylene chloride (or dichloromethane), diethyl ether, methanol, ethanol, water, pyridine and the like]. Unless specified to the contrary, the solvents used in the reactions of the present invention are inert organic solvents.

As used herein "polar solvent" refers to solvents which may be either water-immiscible, such as halogenated hydrocarbons, e.g., methylene chloride, chloroform, etc., or water-miscible, such as acetonitrile, tetrahydrofuran, dimethoxyethane, etc., or alkanols, e.g., methanol, ethanol, isopropanol, etc., or water.

As used herein "nonpolar solvent" refers to solvents such as aliphatic hydrocarbons, such as pentane, hexane, heptane, etc., or aromatic hydrocarbons such as benzene, toluene, xylene, etc.

As used herein, "leaving group" means an atom or a group, charged or uncharged, that is detachable from an atom in what is considered to be the residual or main part of a molecule, including such leaving groups as, halo, alkyl sulfonates, aryl sulfonates, phosphates, sulfonic acid and sulfonic acid salts.

Unless specified to the contrary, the reactions described herein take place at atmospheric pressure over a temperature range from about 0 °C to about 100 °C, more preferably from about 10 °C to about 50 °C, and most preferably at about room (or "ambient") temperature, e.g., about 20 °C.

Isolation and purification of the compounds and intermediates described herein can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the examples hereinbelow. However, other equivalent separation or isolation procedures can, of course, also be used.

As used herein, the term "treatment" or "treating" means any treatment of a disease in a mammal, including:

(i) preventing the disease, that is, causing the clinical symptoms of the disease not to develop;

(ii) inhibiting the disease, that is, arresting the development of clinical symptoms; and/or

(iii) relieving the disease, that is, causing the regression of clinical symptoms.

As used herein, the term "effective amount" means a dosage sufficient to provide treatment for the disease state being treated. This will vary depending on the patient, the disease and the treatment being effected. See, for example, U.S. Patent 4,051,141 to Castaigne (the pertinent portions of which are incorporated herein by reference), for a detailed description of the anti-inflammatory activity, vaso-dilator activity, and inhibitory activity on blood platelet aggregation of the 4,5,6,7-thieno[3,2-c]pyridine derivatives made according to the present invention, as well as the description of toxicological and pharmacological investigations therefor.

Synthesis of the Compounds of Formulae I, II, and IV

The compounds of Formulae I, II, and IV are synthesized as described with reference to Reaction schemes 1 to 5.

Reaction scheme 1 illustrates the conversion of suitable derivatives of 2-(2′-thienyl)ethanol to-form 2-(2′-thienyl)ethylamine.

Reaction scheme 2 illustrates an alternative synthetic route for the conversion of suitable derivatives of 2-(2′-thienyl)ethanol to form 2-(2′-thienyl)ethylamine.

Reaction scheme 3 illustrates the conversion of 2-(2′-thienyl)ethylamine to its carbamic acid salt and the reconversion to the free amine.

Reaction Scheme 4 illustrates the conversion of 2-(2′-thienyl)ethylamine to 4,5,6,7-thieno[3,2-c]pyridine derivatives by N-benzylation or N-benzoylation of 4,5,6,7-tetrahydrothieno-[3,2-c]pyridine.

Reaction Scheme 5 illustrates an alternate synthetic route for the conversion of 2-(2′-thienyl)ethylamine to 4,5,6,7-thieno[3,2-c]pyridine derivatives by N-benzylation or N-benzoylation of 2-(2′-thienyl)ethylamine followed by cyclization.

Preparation of 2-(2′-Thienyl)ethylamine

2-(2′-Thienyl)ethanol and its suitable derivatives, e.g., benzenesulfonate and methanesulfonate, are prepared according to the procedure of Braye, U.S. Patent No. 4,127,580. In addition, 2-(2′-thienyl)ethanol is commercially available from Henley Chemicals, 50 Chestnut Ridge Road, Montvale, New Jersey, 07645.

2-(2′-Thienyl)ethyl sulfonates may be prepared according to procedures well known to those skilled in the art. The most frequently used procedure for the preparation of sulfonates (sulfonic esters) involves reacting the corresponding sulfonyl halides with alcohols in pyridine or a suitable solvent in the presence of a base.

Sulfonyl halides such as benzene sulfonyl chloride and methanesulfonyl chloride are commercially available from inter alia Aldrich Chemical Company, Milwaukee, Wisconsin and Fluka Chemical Corporation, Ronkonkoma, New York.

For the preparation of benzene sulfonates and methane sulfonates see also Fieser and Fieser, Reagents for Organic Synthesis, 1967, pages 46-47 and 662-664 resp.

Ammonia gas of sufficient purity, anhydrous grade, is commerically available from Matheson Gas products, P.O. Box 85, East Rutherford, New Jersey, 07073.

Metal salts, useful in the process of the present invention, are represented by the formula M-Y, where M is selected from Na, Li, K, Mg, and Zn, and Y is halo or a carbonate ($CO_3^{-2}$) anion. These salts are commercially available, e.g., from Aldrich Chemical Company, Inc. 940 West Saint paul Avenue, Milwaukee, Wisconsin 53233. $MgI_2$ is commercially available from Hudson Laboratories, 13923 Old Dixie Highway, Hudson, Fl 33567.

**Reaction Scheme 1**

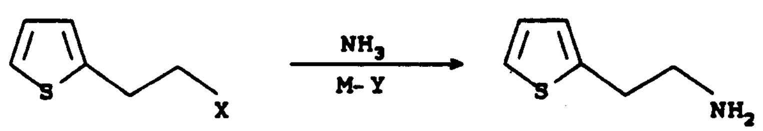


(1) (I)

As illustrated in Reaction Scheme 1, a suitably functionalized derivative of 2-(2′-thienyl)ethanol, i.e., a compound of Formula 1 where X is alkyl sulfonate, aryl sulfonate or halogen, preferably 2-(2′-thienyl)ethyl benzenesulfonate or 2-(2′-thienyl)ethyl methanesulfonate, and about 1 molar equivalent of a metal salt of formula M-Y is dissolved in a solvent (e.g., a polar solvent such as tetrahydrofuran, water, methanol or ethanol, preferably methanol) is placed in a pressurizable reactor. The pressure reactor is rapidly pressurized to about 25 to 150 psi, preferably about 70 to 90 psi with ammonia gas while keeping the internal temperature at about 45 to 60 °C, preferably 50 to 55 °C, and the solution is stirred for 2 to 20 hours, preferably 12 to 16 hours, under ammonia gas.

Alternatively, the functionalized derivative of 2-(2′-thienyl)ethanol, i.e., a compound of Formula 1 where X is alkyl sulfonate, aryl sulfonate or halogen, preferably 2-(2′-thienyl)ethyl benzenesulfonate or 2-(2′-thienyl)ethyl methanesulfonate, can be added into the pressurized reactor using a positive displacement pump. This is preferable on larger scales.

The contents are made acidic, preferably to about pH 2 to 3, and extracted, preferably twice, with a nonpolar solvent such as toluene, or a polar solvent, preferably methylene chloride. The aqueous layer is collected and made basic, preferably to about pH 10 to 12, and extracted, preferably 2 to 3 times, with a nonpolar solvent (e.g., toluene), or a polar solvent, preferably methylene chloride. The organic layers

containing the amine are combined and dried over a dessicant agent, preferably sodium sulfate, and concentrated to give the desired 2-(2′-thienyl)ethylamine as an oil.

Alternatively, water is added to the reaction mixture and the mixture is extracted three times with a nonpolar solvent such as toluene, or a polar solvent, preferably methylene chloride, and dried over $Na_2SO_4$. The solvent is removed under reduced pressure, and the oil is purified by distillation (boiling point 90-95 °C at 4.5mm/Hg) or by precipitation as its carbamic acid salt as described below.

**Reaction Scheme 2**

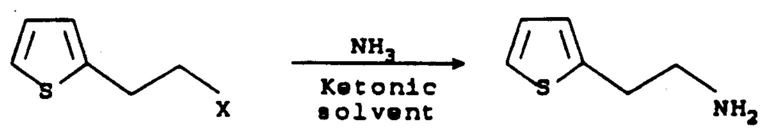


(1) (I)

Alternate Preparation of Formula I

An alternative preparation of the compounds of Formula I is illustrated above in Reaction Scheme 2, where a pressure reactor is charged with a suitably functionalized derivative of 2-(2′-thienyl)ethanol, i.e., a compound of Formula 1 where X is alkyl sulfonate, aryl sulfonate or halogen, preferably 2-(2′-thienyl)ethyl methanesulfonate, and a ketonic solvent, such as an alkyl ketone, e.g., acetone, methylethyl ketone, diethylketone, cyclohexanone, or aryl ketone, e.g., diphenyl ketone, methylphenyl ketone, most preferably acetone, at a molar excess, preferably at a 2 to 1 ratio (molar equivalents) to starting material. Liquid ammonia is added to the pressure reactor at a molar excess, preferably at a 20 to 1 ratio (molar equivalents) to the methanesulfonate. The pressure reactor is sealed and allowed to warm to about 15 °C to 35 ° C, preferably 25 ° C, without external heating. The mixture is stirred for about 8 to 16 hours, preferably 12 hours. The ammonia is released as a gas. The acetone is removed by distillation, and the contents are refluxed for about 1/2 to 2 hours, preferably 1 hour.

The contents are made basic to about pH 10 to 12 by the addition of solid NaOH. The mixture is extracted three times with a polar or nonpolar solvent (e.g., methylene chloride, toluene). The organic layer is dried over a dessicating agent, preferably sodium sulfate. The solvent is removed under reduced pressure.

Alternatively, the tertiary amine by-product can be removed by first making the contents acidic, e.g., upon the addition of HCl, the tertiary amine precipitates out as the HCl salt and is removed by filtration. The contents are then made basic to about pH 10 to 12, e.g., by the addition of solid NaOH, followed by the extraction procedure described above.

Preparation of 2-(2′-Thienyl)ethylamine Carbamic Acid Salt

In a presently preferred process, the 2-(2′-thienyl)-ethylamine prepared as described above, is converted to the carbamic acid salt for purification and to facilitate storage and handling. The crystals are substantially more stable than the 2-(2′thienyl) ethylamine, more specifically, the crystals are resistant to oxidation on exposure to air.

Reaction Scheme 3

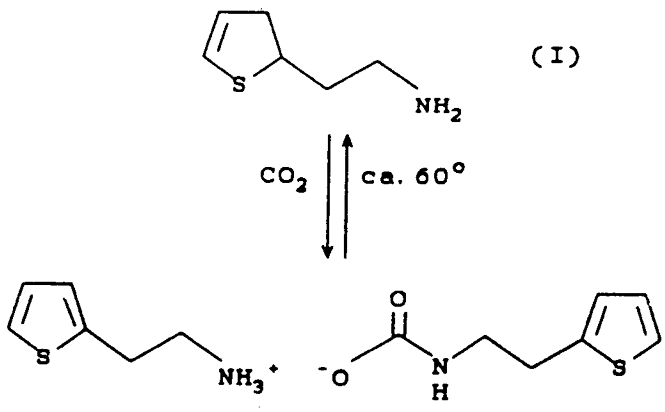


(II)

In order to prepare the carbamic acid salt (II) , as illustrated in Reaction Scheme 3, 2-(2'-thienyl)-ethylamine (I) is dissolved in an inert solvent, preferably toluene or an inert solvent mixture, such as hexane/toluene (ratio from 1:1 to 5:1) and cooled, preferably to about 0 to 10 ° C. The solution is contacted with $CO_2$ (gas or solid) for about one-half to 3 hours, preferably 1 to 2 hours. The carbamic acid salt of the amine precipitates out of solution and is collected by filtration and dried under vacuum at about 20 to 35 ° C, preferably 25 to 30 ° C.

2-(2'-Thienyl)ethylamine is readily recovered from its solid carbamic acid salt and is suitable, without further purification and/or isolation, for use in the synthesis of 4,5,6,7-thieno[3,2-c]pyridine derivatives, particularly ticlopidine, for example as described below.

Reconversion of Carbamic Acid Salt (II) to 2-(2'-Thienyl)ethylamine (I)

The carbamic acid salt (II) is reconverted to the free amine by dissolving it in water and warming the solution to about 60 to 70 ° C. $CO_2$ evolution begins at about 60 ° C. When the conversion is complete (evolution of $CO_2$ ceases) the solution is allowed to cool to room temperature. The solution is then made basic, preferably to about pH 10 to 12, and extracted with a polar or nonpolar solvent, such as methylene chloride or toluene. The extracts are combined and dried over a dessicant, preferably $Na_2SO_4$, and concentrated under reduced pressure to give the free amine, 2-(2'-thienyl)ethylamine, as an oil.

## Reaction Scheme 4

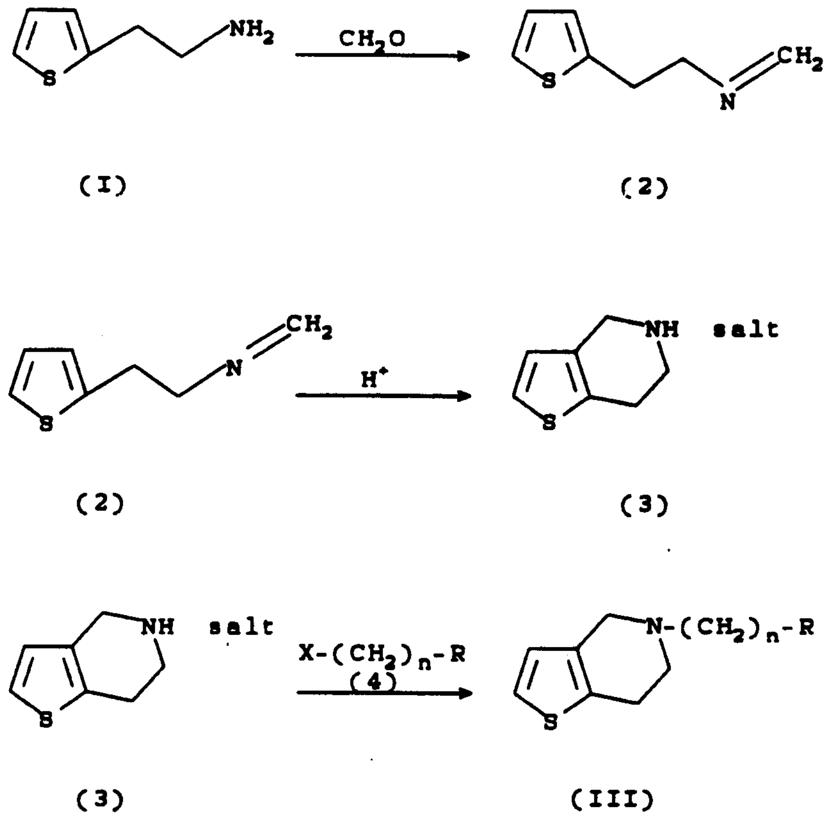


(I) (2)

(2) (3)

(3) (III)

wherein:

X is halogen;

n is 1 or 2;

R is a phenyl or benzoyl radical, optionally substituted with 1 to 3 halogen atoms or alkyl having 1 to 6 carbon atoms, alkoxy having 1 to 6 carbon atoms, or nitro.

Preparation of 4,5,6,7-Tetrahydrothieno[3,2-c]pyridine

4,5,6,7-Tetrahydrothieno[3,2-c]pyridine can be prepared according to the procedure of Gronowitz et al. [Arkiv Kemi, 13(19), 217-227 (1970)] incorporated herein by reference.

As illustrated in Reaction Scheme 4, a slight molar excess of formaldehyde (e.g., a 37% aqueous solution) is added dropwise with stirring to 2-(2′-thienyl)ethylamine (I). The reaction mixture is stirred for about 1 to 5 hours, preferably about 3 hours, at the reflux temperature of the solvent used. After cooling to room temperature, the product is extracted, e.g., into toluene (or another solvent such as methylene chloride, chloroform, or ethyl acetate), washed and concentrated in vacuo to give the formimine of 2-(2′-thienyl)ethylamine (90 to 95% yield of the compound of Formula 2).

The formimine (2) is shaken with a dilute solution of an aqueous acid (such as hydrochloric acid or sulfuric acid); or a solution of formimine (2), dissolved in an organic solvent such as tetrahydrofuran or toluene, preferably tetrahydrofuran, is shaken with an organic acid (such as formic acid, oxalic acid, p-toluenesulfonic acid or methanesulfonic acid, preferably methanesulfonic acid) for 3 to 10 hours, preferably about 6 hours. The mixture is then basified (e.g., with NaOH) and extracted, e.g., with methylene chloride (or another solvent). The extracts are washed and concentrated in vacuo to give 4,5,6,7-tetrahydrothieno [3,2-c]pyridine (80 to 90% yield of the compound of Formula 3).

Preparation of Formula III

Still referring to Reaction Scheme 4, a solution of 4,5,6,7-tetrahydrothieno[3,2-c]pyridine (3) in a solvent (e.g., a polar solvent, such as tetrahydrofuran, methylene chloride or acetonitrile, preferably tetrahydrofuran) is added to a molar excess of a suspension of a base (e.g., a metal hydride, such as lithium hydride, 50% sodium hydride, or potassium hydride, preferably 50% sodium hydride) in the same or a similar solvent. The mixture is stirred at a temperature of about 15 to 30 °C, preferably about room temperature, for about 10 minutes to about 2 hours, preferably about 30 minutes. A slight molar excess (e.g., about 1.05 to 1.1) of an optionally substituted phenalkyl or phenacyl halide of Formula 4 is added (for example the halides of Formula 4 in U.S. Patent No. 4,051,141, such as 4-methoxybenzyl chloride, phenacyl bromide, or preferably o-chlorobenzyl chloride)].

After stirring at about room temperature, for about 1 to 2 hours, preferably about 90 minutes, the mixture is heated, preferably to the reflux temperature of the solvent used. Another solvent (e.g., toluene; xylene, or ether, preferably toluene) is added and the mixture is further refluxed for about 2 to 48 hours, preferably for about 10 to 30 hours, most preferably about 20 hours. The mixture is then cooled to about room temperature and acidified (e.g, with dilute hydrochloric acid or acetic acid, preferably hydrochloric acid).

The organic layer is separated, and the aqueous layer is extracted (e.g., with toluene, methylene chloride, ethyl acetate, or isopropyl acetate, preferably toluene). The combined aqueous layers are basified (e.g., with aqueous NaOH or solid NaOH) to a pH of about 12 to 14. The product is extracted, e.g., into methylene chloride (or another solvent). The extracts are washed (e.g., with water and optionally with a salt solution, e.g., a sodium chloride solution), dried and then concentrated in vacuo to give a compound of Formula III.

When the compound of Formula 4 is o-chlorobenzyl chloride, ticlopidine free base is formed (about 80% yield) as a light yellow oil.

Phase Transfer Alkylation

Alternatively, the alkylation can be performed under phase transfer conditions, e.g., as described in GB 2,166,730, the pertinent parts of which are incorporated herein by reference. The 4,5,6,7-tetrahydrothieno-[3,2-c]pyridine (3) and compound of Formula 4 (preferably o-chlorobenzyl chloride) are dissolved in a solvent system (preferably an aqueous organic two-phase solvent system, the organic phase of which is immiscible with water, e.g., hydrocarbons such as benzene, toluene and xylene, and ethers such as isopropyl ether and diethyl ether, preferably toluene) combined with a phase transfer catalyst [e.g., a quaternary ammonium salt, such as trimethylbenzylammonium hydroxide, trimethylbenzylammonium hydrogen sulfate, tetra-n-butylammonium chloride, trioctylmethylammonium chloride, triethylbenzylammonium chloride or tert-butylammonium iodide ("TBAI"), or a phosphonium salt, such as tetrabutylphosphonium chloride, or a crown ether, such as 18-crown-6 or dibenzo 18-crown-6, preferably TBAI] in the presence of a base (e.g., sodium hydroxide, potassium hydroxide, lithium hydroxide, potassium carbonate, sodium carbonate, or sodium hydride, preferably sodium hydroxide) and stirred for about 24 to 72 hours, preferably about 40 hours, at room temperature. The product (about 75% of theoretical yield) is separated, concentrated, and purified by the usual means.

Preferred Alkylation

In a preferred alkylation procedure, 4,5,6,7-tetrahydrothieno[3,2-c]pyridine in a solvent (e.g., a polar solvent such as tetrahydrofuran, methylene chloride or acetonitrile, preferably 5 to 15% wet tetrahydrofuran) and a compound of Formula 4 (preferably o-chlorobenzyl chloride) are added to a molar excess of a base (e.g., potassium carbonate, sodium carbonate, or lithium carbonate, preferably potassium carbonate) that has been wetted with water (about 5 to 15%, preferably about 10% of the volume charge). The reaction mixture is refluxed until disappearance of the starting materials is confirmed by tlc (about 8 to 42 hours, preferably about 18 to 24 hours). The solvent is removed (by vacuum or by displacement with another solvent such as toluene), and the product is washed with water and then concentrated in vacuo. Using the preferred compound of Formula 4, ticlopidine free base (Formula IV) is formed (about 90 to 95% yield) as a light yellow oil.

Reaction Scheme 5

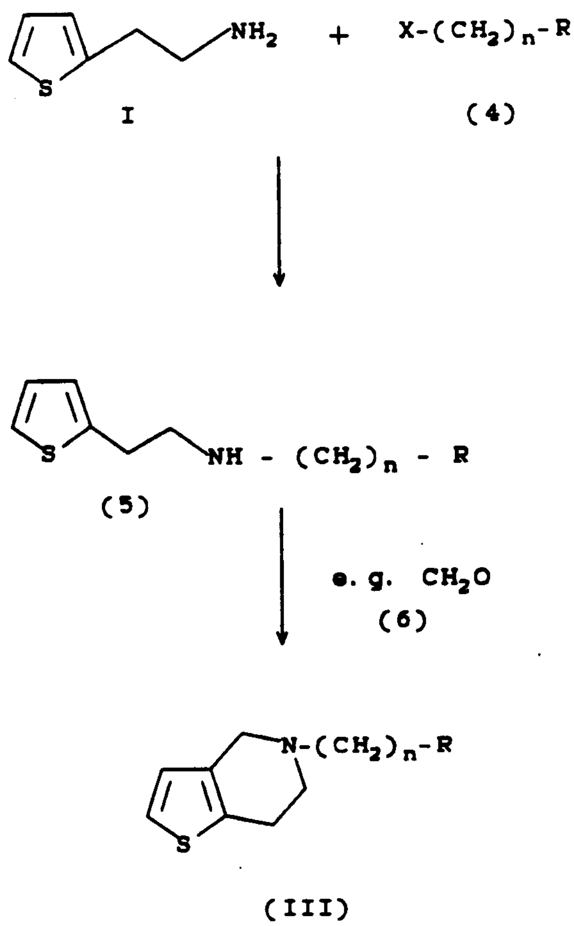


wherein:

X is halogen

n is 1 or 2

R is a phenyl or benzoyl radical, optionally substituted with 1 to 3 halogen atoms or alkyl having 1 to 6 carbon atoms, alkoxy having 1 to 6 carbon atoms, or nitro.

Alternate Preparation of Formula III

Alkylation of 2-(2′-Thienyl)ethylamine, Followed by Cyclization

Another preparation of the compounds of Formula III is illustrated above in Reaction Scheme 5. A compound of Formula I, prepared as described above with reference to Reaction Scheme 1, is reacted with a compound of Formula 4 under the conditions described above to give the secondary amine of Formula 5. Compound (5) is then cyclized by reacting it with a compound identified in Reaction Scheme 5 as Formula 6 (i.e., formaldehyde, paraformaldehyde, trioxane or a compound of Formula 6 of U.S. Patent No. 4,174,448, such as dimethoxymethane) to give a compound of Formula III under the conditions described in U.S. Patent No. 4,174,448, the pertinent portions of which are incorporated herein by reference.

Preparation of Acid Addition Salts of Formula III

The compounds of Formula III may be converted to the corresponding acid addition salts. The conversion is accomplished by treatment with a stoichiometric amount of an appropriate acid, such as hydrochloric acid, hydrobromic acid, sulfuric acid, methanesulfonic acid, or the like (for tyclopidine the

preferred acid is hydrochloric acid). Typically, the free base is dissolved in a polar organic solvent such as ethanol or methanol, and the acid is added in water, ethanol or methanol. The temperature is maintained at 0° to 50°C. The resulting salt precipitates spontaneously or may be brought out of solution with a less polar solvent (such as toluene, ether, or ethyl acetate, preferably toluene).

Preferably, for making ticlopidine hydrochloride, about 1.3 equivalents of hydrogen chloride gas is bubbled into isopropanol, which is then added slowly with stirring to ticlopidine free base in toluene, maintaining the temperature below about 40°C during the addition. The stirring is continued for about 30 to 90 minutes, preferably about 1 hour at about 45 to 50°C, followed by cooling to about 5 to 10°C for about 30 to 90 minutes, preferably about 1 hour. The ticlopidine hydrochloride precipitates and is isolated (e.g., by centrifugation or filtration), digested (e.g., with toluene and isopropanol, or preferably with acetone), dried (e.g., at about 65 to 70°C under vacuum), and recrystallized from a lower alkanol (e.g., methanol, ethanol or isopropanol).

Free Bases from Acid Addition Salts

The acid addition salts of the compounds of Formula III may be converted to the corresponding free bases by treating them with an excess of a suitable base, such as ammonia or sodium bicarbonate, typically in the presence of aqueous solvent, and at a temperature of 0° to 50°C, preferably 20 to 30°C. The free base is isolated by conventional means, such as extraction with an organic solvent.

Preferred Processes and Last Steps

A preferred process for making 2-(2′-thienyl)ethylamine comprises reacting a suitably functionalized derivative of 2-(2′-thienyl)ethanol, more preferably the benzenesulfonate or methanesulfonate, with ammonia gas in the presence of a metal salt, preferably NaBr.

Another preferred process for making 2-(2′-thienyl)ethylamine comprises reacting a suitably functionalized derivative of 2-(2′-thienyl)ethanol, more preferably the benzenesulfonate or methanesulfonate, with liquid ammonia in an alkyl ketone, preferably, acetone.

A preferred process for making ticlopidine comprises the steps of:

a) reacting a suitably functionalized derivative of 2-(2′-thienyl)ethanol with ammonia gas in the presence of a metal salt to give 2-(2′-thienyl)ethylamine;

b) reacting said 2-(2′-thienyl)ethylamine with formaldehyde to give the formimine;

c) cyclizing said formimine by treating it with an aqueous mineral acid to form 4,5,6,7-tetrahydrothieno-[3,2-c]pyridine;

d) alkylating said 4,5,6,7-tetrahydrothieno-[3,2-c]pyridine by reacting it with 2-chlorobenzyl chloride to give ticlopidine free base under either:

(i) phase transfer conditions, or

(ii) preferably, by reflux with a base, most preferably potassium carbonate, using conventional alkylation conditions; and

e) converting ticlopidine free base to a pharmaceutically acceptable acid addition salt, preferably the hydrochloride salt.

Further preferred are the processes wherein the 2-(2′-thienyl)ethylamine, prepared as in step (a) above, is converted to its carbamic acid salt for purification, storage, transportation and/or further reacting, or is suitably converted back to 2-(2′-thienyl)ethylamine for subsequent use in step (b) as described above.

Another preferred process for making ticlopidine comprises the steps of:

a) reacting a suitably functionalized derivative of 2-(2′-thienyl)ethanol with liquid ammonia in the presence of a ketonic solvent to give 2-(2′-thienyl)ethylamine;

b) reacting said 2-(2′-thienyl)ethylamine with 2-chlorobenzyl chloride to give N-(2-chlorobenzyl)-2-(2′-thienyl)ethylamine;

c) cyclizing said N-(2-chlorobenzyl)-2-(2′-thienyl)ethylamineby treating it with formaldehyde to form ticlopidine free base; and

e) converting ticlopidine free base to a pharmaceutically acceptable acid addition salt, preferably the hydrochloride.

Further preferred are the processes wherein the 2-(2′-thienyl)ethylamine, prepared as in step (a) above, is converted to its carbamic acid salt for purification, storage, transportation and/or further reacting, or is suitably converted back to 2-(2′-thienyl)ethylamine for subsequent use in step (b) as described above.

Preferred Compounds

The preferred compounds made by the processes of the invention are 2-(2′-thienyl)ethylamine, 2-(2′-thienyl)ethylamine carbamic acid salt and 5-(2-chlorobenzyl)-4,5,6,7-tetrahydrothieno-[3,2-c]pyridine, which is also known as ticlopidine (free base). Particularly preferred is ticlopidine hydrochloride.

**EXAMPLES**

The following preparations and examples are given to enable those skilled in the art to more clearly understand and to practice the present invention. They should not be considered as limiting the scope of the invention, but merely as being illustrative and representative thereof.

Example 1

Preparation of 2-(2′-Thienyl)ethylamine (TEA)

1A. Preparation from 2-(2′-Thienyl)ethylbenzenesulfonate

A 1 liter Parr pressure reactor was charged with 70 g of 2-(2′-thienyl)ethyl benzenesulfonate, 250 ml of methanol, 50 ml of concentrated aqueous ammonium hydroxide and 26.4g of sodium bromide (sodium bromide is about 90% soluble after a few minutes of stirring). The reactor was pressurized to 80 psi with ammonia gas while maintaining the internal temperature at 50 °C. The reaction mixture was stirred overnight. The ammonia gas was then released, the contents were acidified to about pH 2 to 3 and extracted twice with methylene chloride. The aqueous layer was made basic to about pH 10 to 12 with sodium hydroxide pellets and extracted three times with methylene chloride. The combined organic layers, containing the TEA, were dried over sodium sulfate and concentrated to give 33.9 g of an oil that contained about 55-60% of TEA as determined by gas chromatography (b.p. 115 - 130 °C./20 - 22 mm Hg).

1B. Preparation of TEA Carbamic Acid Salt

The 2-(2′-thienyl)ethylamine obtained in 1A above (33.9 g of oil, ca. 55-60% pure) was dissolved in hexane/toluene (4:1) (ca. 500 ml) and cooled to 0 °C. Carbon dioxide gas was bubbled through the solution for about 1 hour at 0 to 10 °C with stirring. The precipitated solids were collected by filtration and dried under vacuum at 30 °C.

Following the above procedure, there was obtained 25.7 g of the carbamate salt (about 66% overall yield based on preparations 1A and 1B).

**Elemental analysis:**

| | % Calculated | % Found |
|---|---|---|
| C | 52.35 | 52.39 |
| H | 6.09 | 6.04 |
| N | 9.11 | 9.40 |
| S | 21.40 | 21.47 |

Example 2

2A. Alternative Preparation from 2-(2′-Thienyl)ethyl-methanesulfonate

A 1 liter Parr pressure reactor was charged with 51.7 g of 2-(2′-thienyl)ethyl methanesulfonate, 25.9 g of NaBr, 200 ml of methanol, 100 ml of concentrated aqueous ammonium hydroxide and 25.9 g of sodium

bromide. The contents were cooled to about 10 ° C and the reactor was pressurized to 80 psi with ammonia gas. The reactor temperature was brought up to 50 ° C while maintaining 80 psi of ammonia. The reaction mixture was stirred for 16 hours. The ammonia gas was then released and the contents were acidified to about pH 2 to 3 and extracted twice with methylene chloride. The aqueous layer was made basic to about pH 10 to 11 with sodium hydroxide pellets and extracted three times with methylene chloride. The combined organic layers, containing the TEA, were dried over sodium sulfate and concentrated to give 39.3 g of an oil. The TEA oil was ca. 70% pure by gas chromatography (b.p. 115 to 130 ° C, 20 to 22 mm Hg).

2B. Preparation of the Carbamic Acid Salt

The TEA oil (39.3 g, ca. 70% pure) obtained in 2A above was dissolved in hexane/toluene (4:1) and cooled to 0 to 5 ° C. Carbon dioxide gas was bubbled through the solution for 1 hour with stirring. The precipitated solids were collected by filtration and dried under vacuum at 30 ° C.

Following the above procedure, there was obtained 30.1 g of the carbamate salt, at an internal standard of purity of 99.7% (about 80% overall yield based on preparations 2A and 2B).

Example 3

3A. Alternative Preparation of 2-(2′-Thienyl)ethylamine (TEA) using liquid ammonia

A pressure reactor was charged with 189 mmoles (35.7 g) of 2-(2′-thienyl)ethyl methanesulfonate and 378 mmoles (22 g) of acetone. The contents were cooled below -33 ° C and 150 mL of liquid ammonia was added. The reactor was then sealed and the contents were allowed to warm to 25 ° C without external heating and the mixture was stirred overnight. The ammonia was released and the contents were transferred to a flask. The acetone was removed by distillation at up to 85 ° C. The residue was refluxed for 1 hour and was then made basic to pH 10 to 11 by adding solid NaOH. The reaction mixture was extracted with methylene chloride, the organic layer was separated and dried over $Na_2SO_4$. The solvent was stripped off under reduced pressure. Kugelrohr distillation gave 18.7 g of 2-(2′-thienyl)ethylamine (78% yield of 90.2% purity). NMR($CDCl_3$)δ: 6.8-7.2(m,3H),2.92(br.s,4H), 1.28(br.s,2H).

3B. Alternative Preparation of 2-(2′-Thienyl)ethylamine Using Liquid Ammonia

By following the procedure of Example 3A and substituting diphenyl ketone for acetone 2-(2′-Thienyl)-ethylamine is obtained in similar yield and purity.

3C. Preparation of the 2-(2′-Thienyl)ethylamine Carbamic Acid Salt

A solution of 2-(2′-thienyl)ethylamine (10g) in toluene/hexane (1:1) (100 ml) was cooled to 0 ° C. Solid carbon dioxide (dry ice) was added in small portions with stirring over a period of 1 hour. The temperature of the reaction mixture was kept between 0 ° to -60 ° during the addition of solid $CO_2$. The temperature of the reaction mixture was then allowed to rise to 0 °. The crystals were collected by filtration and dried under vacuum at 30 ° C. 10.6 g of 2-(2′-Thienyl)ethylamine carbamic acid salt was obtained (94%).

Example 4

Preparation of Ticlopidine

4A. Formimine of 2-(2′-Thienyl)ethylamine

A 37% aqueous formaldehyde solution (2.7 g, 0.033 mole) was added dropwise with stirring to 2-(2′-thienyl)ethylamine (3.4 g, 0.027 mole) obtained, e.g., by warming 4 g (0.0135 mole) of carbamic acid salt in 8 ml of $H_2O$ at 65 ° C (1 mole of carbamate contains 2 moles of amine). The reaction mixture was stirred for three hours at reflux. After cooling to room temperature, the product was extracted into toluene (2 x 50 ml); the toluene extracts were washed with water (50 ml) and concentrated to give 3.4 g (91%) of the formimine of 2-(2′-thienyl)ethylamine.
Reported NMR ($CDCl_3$) δ: 7.2-6.8 (m,3H), 3.46 (s,2H), 3.0-2.7 (m,4H).

4B. 4,5,6,7-Tetrahydrothieno[3,2-c]pyridine

The formimine of 2-(2′-thienyl)ethylamine (3.4 g), prepared in Example 4A above, was shaken with 7 ml of 6N hydrochloric acid for six hours. The mixture was basified with 60 ml of 1N sodium hydroxide solution and extracted with 3 x 70 ml methylene chloride. The methylene chloride extracts were washed with water (1 x 50 ml) and concentrated to give 3.4 g of crude 4,5,6,7-tetrahydrothieno-[3,2-c]pyridine (nearly quantitative yield).
Reported NMR ($CDCl_3$) δ: 7.06 (d,1H), 6.72 (d,1H), 3.9 (br.s,2H), 3.2-2.7 (m,4H), 2.10 (br.s,-NH).

4C. Ticlopidine Free Base

To a suspension of sodium hydride (0.42 g, 8.6 mmole) in tetrahydrofuran (5.0 ml) was added a solution of 4,5,6,7-tetrahydrothieno[3,2-c]pyridine (1.0 g, 7.2 mmole), prepared, for example, as described in Example 4B, in tetrahydrofuran (10 ml). The mixture was stirred under a nitrogen atmosphere at room temperature for 30 minutes and o-chlorobenzyl chloride (1.74 g, 10.8 mmole) was added. After stirring at room temperature for 90 minutes, toluene (15 ml) was added and the mixture was heated under reflux for 15 to 20 hours. Disappearance of starting material was confirmed by tlc. The mixture was then cooled to room temperature and acidified with 40 ml 1N hydrochloric acid. The organic layer was separated and the aqueous layer was extracted with 50 ml of toluene. The aqueous layer was separated and basified with dilute aqueous sodium hydroxide to pH 13 to 14. The product was extracted into methylene chloride (3 x 40 ml). The methylene chloride extracts were washed (1 x 50 ml water) and (1 x 50 ml salt solution), dried over anhydrous magnesium sulfate and concentrated under reduced pressure to give 1.5 g (80%) of 5-(2-chlorobenzyl)-4,5,6,7-tetrahydro-thieno[3,2-c]pyridine (ticlopidine free base) as a light yellow oil.
Reported NMR ($CDCl_3$) δ: 7.7-7.15 (m,4H), 7.05 (d,1H), 6.65 (d,1H), 3.8 (s,2H), 3.6 (s,2H), 2.85 (br.s,4H).

4D. Ticlopidine Hydrochloride

HCl gas (0.22 g, 0.006 mole) was bubbled into isopropanol (50 ml). The resulting solution was added dropwise to ticlopidine free base (1.5 g, 0.005 mole) in 50 ml toluene, prepared, for example as described in Example 4C, maintaining the temperature below 40 °C during the addition. The reaction mixture was stirred for 1 hour, cooled to about 5 to 10 °C for 1 hour, and the precipitate was separated by centrifugation. An acetone slurry of the precipitate was heated under reflux for 1 hour, and was then cooled to about 5 to 10 °C for 1 hour. The precipitate was separated by centrifugation, dried at 65 to 70 °C under vacuum, and recrystallized from methanol to give 1.58 g (93%) of ticlopidine hydrochloride of Formula IV. (m.p. 206.5 - 207.5 °C).

**Claims**

**1.** A process for making 2-(2'-thienyl)ethylamine comprising reacting a compound of the formula

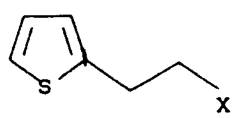

wherein
X is a leaving group,
a) with gaseous ammonia in the presence of a compound of the formula M-Y,
wherein:
M is Na, K, Li, Mg, Zn; and
Y is halide or carbonate
b) with liquid ammonia, and
an alkyl ketone or aryl ketone.

**2.** The process of Claim 1, step (a), wherein M-Y is NaBr.

**3.** The process of Claim 1 wherein the leaving group is halo, alkyl sulfonate, or aryl sulfonate.

4. The process of Claim 1, step (b), wherein the alkyl ketone or the aryl ketone is selected from the group consisting of acetone, diethyl ketone, methylethyl ketone, methylphenyl ketone, diphenyl ketone and cyclohexanone.

5. The process of Claim 4 wherein the alkyl ketone is acetone.

6. A process for the preparation of the carbamate salt of 2-(2'-thienyl)ethylamine of the formula:

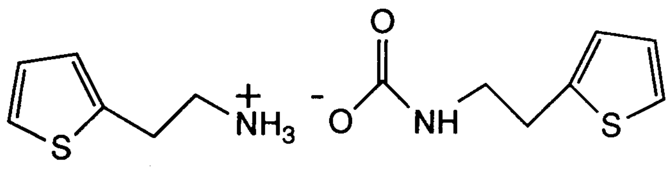

comprising the steps consisting of :

a) reacting 2-(2'-thienyl)ethylamine prepared according to the process of claim 1, previously dissolved in an inert solvent, with $CO_2$, and

b) isolating the carbamate salt of 2-(2'-thienyl)ethylamine.

7. The process of Claim 6 wherein the inert solvent is selected from the group consisting of toluene, benzene, hexane, cyclohexane, heptane, octane, or mixtures thereof.

8. A process according to claim 1, further comprising the steps consisting of :

c) preparing the carbamate salt of 2-(2'-thienyl)ethylamine using the process of claim 6 ; and

d) reconverting said 2-(2'-thienyl)ethylamine carbamic acid salt to 2-(2'-thienyl)ethylamine.

9. A process for the preparation of ticlopidine free base or its addition salt comprising the step consisting of :

a) reacting 2-(2'-thienyl)ethylamine of the formula :

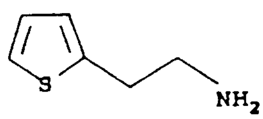

prepared according to the process of claim 1 or 9 with formaldehyde ($CH_2O$) to give the formimine of the formula

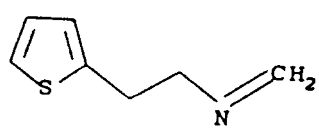

b) cyclizing the formimine by reacting it with aqueous hydrochloric acid to form 4,5,6,7-tetrahydrothieno[3,2-c]pyridine of the formula

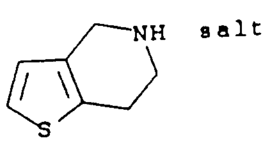

c) alkylating 4,5,6,7-tetrahydrothieno[3,2-c]pyridine by reacting it with 2-chlorobenzyl chloride

(i) under phase transfer conditions, or

(ii) by reflux with a base
to give ticlopidine free base of the formula

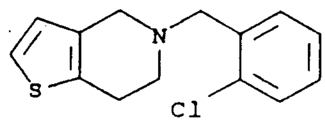

and optionally
d) converting ticlopidine free base to an acid addition salt, preferably the hydrochloride salt.

**10.** A process for the preparation of ticlopidine free base or its addition salt comprising the steps consisting of :
a) reacting 2-(2'-thienyl)ethylamine prepared according to the process of claim 1 or 9 with 2-chlorobenzyl chloride to give N-(2-chlorobenzyl)-2-(2'-thienyl)ethylamine ;
b) cyclizing N-(2-chlorobenzyl)-2-(2'-thienyl)ethylamine by reacting it with formaldehyde to form ticlopidine free base; and optionally
c) converting ticlopidine free base to an acid addition salt, preferably the hydrochloride salt.

**Patentansprüche**

**1.** Verfahren zur Herstellung von 2-(2'-Thienyl)-ethylamin umfassend die Umsetzung einer Verbindung der Formel

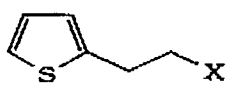

in der
X eine austretende Gruppe bedeutet,
a) mit gasförmigem Ammoniak in Gegenwart einer Verbindung der Formel M-Y,
in der:
M, Na, K, Li, Mg, Zn und
Y Halogenid oder Carbonat bedeuten,
b) mit flüssigem Ammoniak und einem Alkylketon oder einem Arylketon.

**2.** Verfahren nach Anspruch 1, Stufe (a) worin M-Y NaBr ist.

**3.** Verfahren nach Anspruch 1, worin die austretende Gruppe Halogen, Alkylsulfonat oder Arylsulfonat ist.

**4.** Verfahren nach Anspruch 1, Stufe (b), worin das Alkylketon oder das Arylketon aus der Gruppe ausgewählt ist, die aus Aceton, Diethylketon, Methylethylketon, Methylphenylketon, Diphenylketon und Cyclohexanon besteht.

**5.** Verfahren nach Anspruch 4, worin das Alkylketon Aceton ist.

**6.** Verfahren zur Herstellung des Carbamatsalzes von 2-(2'-Thienyl)-ethylamin der Formel:

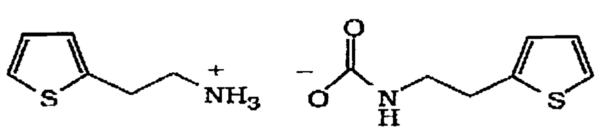

umfassend die Schritte:

a) Umsetzen des gemäß dem Verfahren nach Anspruch 1 hergestellten und zuvor in einem innerten Lösungsmittel gelösten 2-(2'-Thienyl)ethylamins mit $CO_2$ und

b) Isolieren des Carbamatsalzes des 2-(2'-Thienyl)-ethylamins.

**7.** Verfahren nach Anspruch 6, worin das innerte Lösungsmittel aus der Gruppe ausgewählt ist, die aus Toluol, Benzol, Hexan, Cyclohexan, Heptan, Octan oder Mischungen davon besteht.

**8.** Verfahren nach Anspruch 1, weiterhin umfassend die Schritte:

c) Herstellen des Carbamatsalzes von 2-(2'-Thienyl)-ethylamin unter Anwendung des Verfahrens nach Anspruch 6; und

d) Umwandeln des 2-(2'-Thienyl)-ethylamin-carbamidsäuresalzes in 2-(2'-Thienyl)-ethylamin.

**9.** Verfahren zur Herstellung von Ticlopidin in Form der freien Base oder seines Additionssalzes, umfassend die Schritte:

a) Umsetzen des nach dem Verfahren des Anspruchs 1 oder 9 hergestellten 2-(2'-Thienyl)-ethylamins der Formel:

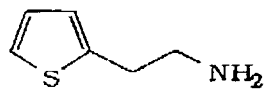

mit Formaldehyd ($CH_2O$) zur Bildung des Formimins der Formel:

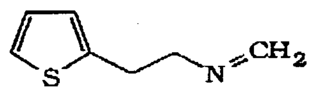

b) Cyclisieren des Formimins durch Umsetzen mit wäßriger Chlorwasserstoffsäure zur Bildung von 4,5,6,7-Tetrahydrothieno[3,2-c]pyridin der Formel:

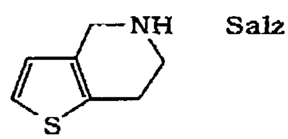

c) Alkylieren des 4,5,6,7-Tetrahydrothieno[3,2-c]pyridins durch Umsetzen mit 2-Chlorbenzylchlorid

(i) unter Phasentransferbedingungen oder

(ii) am Rückfluß in Gegenwart einer Base

zur Bildung von Ticlopidin in Form der freien Base der Formel:

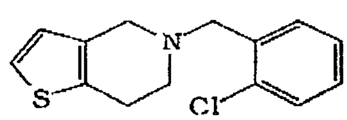

und gegebenenfalls

d) Umwandeln von Ticlopidin in Form der freien Base in ein Säureadditionssalz, vorzugsweise das Hydrochloridsalz.

10. Verfahren zur Herstellung von Ticlopidin in Form der freien Base oder seines Säureadditionssalzes umfassend die Schritte:
   a) Umsetzen des nach dem Verfahren von Anspruch 1 oder 9 hergestellten 2-(2'-Thienyl)-ethylamins mit 2-Chlorbenzylchlorid zur Bildung von N-(2-Chlorbenzyl)-2-(2'-Thienyl)-ethylamin;
   b) Cyclisieren des N-(2-Chlorbenzyl)-2-(2'-thienyl)-ethylamins durch Umsetzen mit Formaldehyd unter Bildung von Ticlopidin in Form der freien Base; und gegebenenfalls
   c) Umwandeln von Ticlopidin in Form der freien Base in ein Säureadditionssalz, vorzugsweise das Hydrochloridsalz.

## Revendications

1. Procédé pour préparer la 2-(2'-thiényl)éthylamine, comprenant la réaction d'un composé de formule :

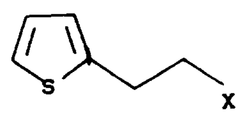

dans laquelle
   X est un groupe labile,
   a) avec de l'ammoniac gazeux en présence d'un composé de formule M-Y,
      dans laquelle :
      M est Na, K, Li, Mg ou Zn ; et
      Y est halogénure ou carbonate,
   b) avec de l'ammoniac liquide et une alkylcétone ou une arylcétone.

2. Procédé selon la revendication 1, étape (a), où M-Y est NaBr.

3. Procédé selon la revendication 1, où le groupe labile est halogéno, alkylsulfonate ou arylsulfonate.

4. Procédé selon la revendication 1, étape (b), où l'alkylcétone ou l'arylcétone est choisie dans le groupe constitué par l'acétone, la diéthylcétone, la méthyléthylcétone, la méthylphénylcétone, la diphénylcétone et la cyclohexanone.

5. Procédé selon la revendication 4, où l'alkylcétone est l'acétone.

6. Procédé pour la préparation du sel carbamate de la 2-(2'-thiényl)éthylamine de formule :

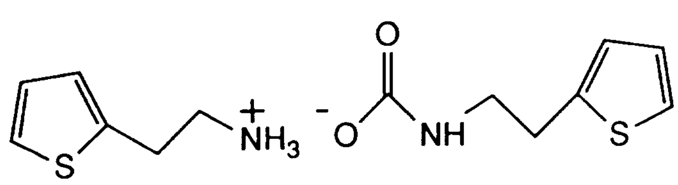

comprenant les étapes consistant à
   a) faire réagir la 2-(2'-thiényl)éthylamine préparée selon la procédé de la revendication 1, préalablement dissoute dans un solvant inerte, avec du $CO_2$, et
   b) isoler le sel carbamate de la 2-(2'-thiényl)éthylamine.

7. Procédé selon la revendication 6, où le solvant inerte est choisi dans le groupe constitué par le toluène, le benzène, l'hexane, le cyclohexane, l'heptane, l'octane ou leurs mélanges.

8. Procédé selon la revendication 1, comprenant de plus les étapes consistant à :
   c) préparer le sel carbamate de la 2-(2'-thiényl)éthylamine selon le procédé de la revendication 6 ; et

d) retransformer ledit sel d'acide carbamique de la 2-(2'-thiényl)éthylamine en 2-(2'-thiényl)-éthylamine.

**9.** Procédé pour la préparation de la ticlopidine, sous forme de base libre ou d'un de ses sels d'addition, comprenant les étapes consistant à :

a) faire réagir la 2-(2'-thiényl)éthylamine de formule :

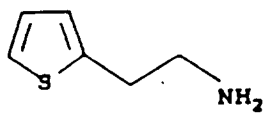

préparée selon le procédé de la revendication 1 ou 9, avec du formaldéhyde ($CH_2O$) pour obtenir la formimine de formule :

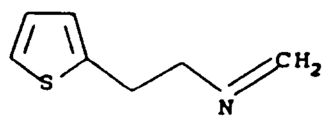

b) cycliser la formimine en la faisant réagir avec de l'acide chlorhydrique aqueux pour former la 4,5,6,7-tétrahydrothiéno [3,2-c]pyridine de formule :

sel de

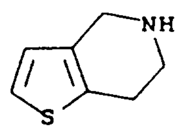

c) alkyler la 4,5,6,7-tétrahydrothiéno[3,2-c]pyridine en la faisant réagir avec le chlorure de 2-chlorobenzyle

(i) dans des conditions de transfert de phase, ou

(ii) par reflux avec une base

pour obtenir la ticlopidine sous forme de base libre de formule :

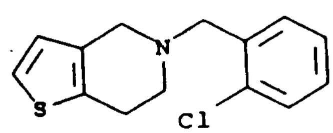

et, éventuellement,

d) transformer la ticlopidine sous forme de base libre en un sel d'addition d'acide, de préférence le chlorhydrate.

**10.** Procédé pour la préparation de la ticlopidine, sous forme de base libre ou d'un de ses sels d'addition, comprenant les étapes consistant à :

a) faire réagir la 2-(2'-thiényl)éthylamine, préparée selon le procédé de la revendication 1 ou 9, avec le chlorure de 2-chlorobenzyle pour obtenir la N-(2-chlorobenzyl)-2-(2'-thiényl)éthylamine ;

b) cycliser la N-(2-chlorobenzyl)-2-(2'-thiényl)éthylamine en la faisant réagir avec le formaldéhyde pour former la ticlopidine sous forme de base libre ; et, éventuellement,

(c) transformer la ticlopidine sous forme de base libre en un sel d'addition d'acide, de préférence le chlorhydrate.